Europäisches Patentamt

European Patent Office

Office européen des brevets

(19)

(11) Publication number: **0 174 958 B1**

# EUROPEAN PATENT SPECIFICATION

(12)

(45) Date of publication of patent specification: **18.12.91**  (51) Int. Cl.⁵: **A61K 31/35, A61K 31/37**

(21) Application number: **85901294.0**

(22) Date of filing: **01.03.85**

(86) International application number:
**PCT/AU85/00035**

(87) International publication number:
**WO 85/03864 (12.09.85 85/20)**

(54) **TREATMENT OF HIGH PROTEIN OEDEMAS BY DIRECT APPLICATION OF BENZO-PYRONES.**

(30) Priority: **01.03.84 AU 3876/84**

(43) Date of publication of application:
**26.03.86 Bulletin 86/13**

(45) Publication of the grant of the patent:
**18.12.91 Bulletin 91/51**

(84) Designated Contracting States:
**CH DE FR GB LI NL**

(56) References cited:
**AU-A- 25 215**
**CH-A- 644 374**
**FR-A- 2 424 026**
**GB-A- 2 052 980**

(73) Proprietor: **LAMORNA INVESTMENTS PROPRI-ETARY LIMITED**
**94 Cambridge Terrace**
**Malvern South Australia(AU)**

(72) Inventor: **CASLEY-SMITH, John Royle**
**97 Seaview Road**
**Tennyson South Australia, 5022(AU)**

(74) Representative: **Silverman, Warren et al**
**HASELTINE LAKE & CO. Hazlitt House 28**
**Southampton Buildings Chancery Lane**
**London WC2A 1AT(GB)**

## Description

This invention relates to the treatment of high-protein oedemas in humans, more particularly relates to the application of benzo-pyrones directly to the skin and/or mucous membranes.

Oedema is an abnormal accumulation of fluid in the tissue spaces or cavities of the body. There are five main factors in the formation of generalised oedema and a sixth which plays an important role in the formation of local oedema. They are:-

1. Permability of the capillary wall

2&3 Colloidal osmotic pressure of the plasma proteins in the blood and tissues

4&5 Hydrostatic pressures in the capillaries and tissues and

6. Lymphatic obstruction.

Thus osdema is an unusual swelling of the tissue due to an excessive amount of fluid and can be the result of various causes. Thus haematoma, or in other words a bruise, is one of the most common oedemas. The causes of oedema fall naturally into four groups corresponding to the four classes of oedema, thus -

1. High-flow low-protein

2. High-flow high-protein

3. Low-flow high-protein, as well as a fourth group of causes of oedema "safety valve insufficiency" which occurs when lymphostasis is superimposed on what would normally be a high flow oedema produced either by excess blood vascular leakage, or the obstruction of a duct of an organ (kidney, pancreas etc.) the results of which are particularly disastrous for the tissues.

High-protein oedemas are very common in all communities. It has been found that one person in three seeks medical attention every year in South Australia, for a condition associated with one. The W.H.O. estimates that 250,000,000 people suffer from lymphoedema and elephantiasis, while 250,000 women in Australia suffer from lymphoedema of the arm, of varying grades of severity, after mastectomy. It is highly probable that the improvement produced by the benzo-pyrone group of drugs, in so many different diseases, is because they have high-protein oedema associated with them. This always causes reduced oxygenation and function of the tissues. If the oedema is reduced, oxygenation and function are improved. While these drugs do not affect the basic diseases, the reduction they produce in associatedoedemas improve the function of the tissues and thus the normal healing processes proceed more expeditiously. Examples of this are: lymphoedema, accidental and surgical trauma, haematomas, pancreatitis, hepatitis and cirrhosis.

It has been found that the group of benzo-pyrones is highly beneficial in the treatment of oedemas, particularly high protein oedemas.

Members of the benzo-pyrone group of drugs have been used since before 1960 in the treatment of high-protein oedemas in man, giving the drugs by intravenous, subcutaneous or intramuscular injection, or orally or rectally. Apart from the use of disodium cromoglycate in tooth-paste and the rectal route via suppositories containing a mixture of coumarin and a rutin derivative, they have never been applied to the skin or mucous membranes.

This has been due to the fact that these drugs are known to be very insoluble, and hence not suitable for application to the skin. However, it has now been discovered that the drugs are sufficiently soluble to be absorbed into the skin and to have a good effect.

Thus there is provided according to the invention use of pharmacologically acceptable benzopyrone drugs in the manufacture of pharmaceutical formulations for topical treatment of high protein oedemas by application to the skin or mucous membranes. Of particular interest is the use of coumarin in a cream and the use of diosmin to treat high-protein oedemas. This use is to be distinguished from that disclosed in FR-A-2 424 026 which proposes the use of certain water-soluble ethers of hydroxy flavones in the treatment of capillary fragility which is one of the causes of high-protein oedemas. These highly water-soluble substances may be administered to the skin as well as by oral, intravenous or intraperitoneal routes. However there is no disclosure of the treatment of any present high-protein oedemas.

The invention includes the use of a mixture of a benzopyrone compound with other drugs with similar or complementary actions, in a pharmaceutical composition designed to be applied to the skin or mucous membranes either by surface application on or by direct surgical implantation or other method of local application in the skin or mucous membrane (e.g. by iontophoresis). Such drugs may be formulated as: a powder, cream, ointment, lotion, spray, paste (including tooth-paste). Penetration of the drug may, but need not, be assisted by the use of a carrier e.g. dimethylsulphoxide.

It has been shown that these drugs can effectively reduce high protein oedemas and they do this by increasing the normal proteolysis in the tissues and (to some extent) by increasing lymphatic function. They are thus effective in all diseases which have high protein oedema as part of their disorders.

While they do not cure any underlying disease, the fact that they reduce the associated high protein oedema means that they relieve much pain and loss of function. In addition all oedemas cause lowered oxygenation and much harm to the tissues. The high protein ones in particular (if prolonged) cause chronic inflammation and thus their removal is of considerable benefit.

The benzo-pyrones are a group of compounds which include a number of sub-groups. They contain the flavonoids and their derivatives, coumarin and its derivatives and a variety of other groups.

It has been found that the benzo-pyrones as noted above are very effective in the treatment of high protein oedemas. It has been found that the use of the benzo-pyrone affects the oedema in four ways:-

1. Excessive protein loss from the blood vessels can be reduced (under certain conditions)

2. Protein and fluid removal by the lymphatics can be increased (under certain conditions)

3. More phagocytosis of protein by cells in the tissues occurs and

4. The intracellular or extracellular, lysis of proteins by cells in the tissues are made greater.

It should be realised however that it may be rare for a benzo-pyrone to have only one action in a particular disease; similarly, it is rare for a particular disease to have only one cause or to derange only one of the body's functions. Thus for example a burn gives a high protein oedema because of the injured blood vessels, but frequently the collecting lymphatics go into spasm producing a super-imposed lymphoedema ie. there is a 'safety valve' insufficiency; treating a burn with a benzo-pyrone may well affect the open blood capillary endothelial junctions, interfere with the mediators of inflammation, alter the permeability of the interstitial tissue, increase the extravascular proteolysis and cause the collecting lymphatics to pump more lymph.

Benzo-pyrones while often opening blood vascular junctions, can greatly reduce blood vascular endothelial damage in certain circumstances. These include preventing the opening of the post capillary venular endothelial junctions, preventing endothelial cells leaving the vessel wall with a consequent huge gap in its lining, and acting as vitamin-P substances when the patient is deficient in this.

It has been shown that benzo-pyrones can increase the pumping capacity of the collecting lymphatics and that they also cause an increased production of urine.

Increased phagocytosis may indeed occur, but the phagocytosed macromolecules are not retained in the tissues. In burns the removal of the protein was considerably increased by coumarin under all conditions. The removal of a non-metabolisable tracer PVP, was slowed by the coumarin in the normal and burnt legs - possibly because phagocytosis was enhanced (with the non-metabolisable PVP being retained in the phagocytes); the PVP was however more rapidly removed with coumarin in the presence of lymphodema - probably because the reduction of oedema reduced the distances it had to travel to reach blood capillaries. The important thing is that the protein removal from the limbs was much more rapid with coumarin. This shows that it is not simply phagocytosed and retained in the phagocytes in the region.

It has also been shown by exclusion that, when these benzo-pyrones reduce high protein oedemas they must increase proteolysis and secondly it was found that the ratios of radio-labelled-protein-fragments, to the labelled protein, increased greatly when treated with coumarin; thirdly that the benzo-pyrones have been shown to induce increased levels of proteases in the oedema fluid, in the whole tissues, and in the plasma and lymph; and fourthly they increase proteolysis by macrophages in vitro and fifthly they lose their ability to reduce high protein oedemas when the macrophages are selectively poisoned.

Benzo-pyrones are effective in the treatment of aphthous ulcers, ulcers from any other cause, haemorrhoids and their complications.

Also the drug can be used in the treatment of inflammation caused by ionising radiations and ultraviolet rays, pancreatitis from any cause, fragility of the blood vessels, and migraine.

It is to be noted that some benzo-pyrones kill bacteria, viruses, intestinal parasites; those which do not, at least do not worsen the conditions (e.g. during infection) - unlike the action of steroids.

Benzo-pyrones have anti-neoplastic activity and may be used to control or destroy, neoplasms - including carcinoma and melanomas, and can be used in allergic conditions - of the skin, mucosa, and deeper organs (autoimmune diseases), and also be used to improve the functioning of the lymphatic system.

It is to be noted that this is only a short list of the conditions associated with high-protein oedemas, any high-protein oedema is deleterious, and that although it may not be a major part of some disease, it should always be treated if possible. It is to the treatment of such oedemas that this invention is directed.

Examples included in the group of benzo-pyrones include coumarin, 7-hydroxy derivatives of coumarin, rutin, troxerutin and diosmin.

In one example a solution of coumarin in polyethylene glycol 400 was prepared and used as a paint on the skin. Preferably a 10% by weight solution is used but solutions of 2.5% to 20% by weight of coumarin in the polyethylene glycol 400 can be used.

In a second example 20% by weight 7 hydroxy coumarin in an inert cream, such as cetomacrosol was prepared and applied. The range of 7 hydroxy coumarin in the carrier cream can vary from 2.5% up to a maximum of 90% by weight.

Thus it can be seen that the use or benzopyrones, particularly coumarin is particularly effective in reducing all forms of oedemas, and the dosage and treatment will vary according to the particular oedema being treated.

However, it is to he noted that the following topical use of benzopyrone drugs, which do not extend to the treatment of high protein oedemas are specifically disclaimed:-

a. The use of sodium cromoglycate in toothpaste or lozenge in the treatment of aphthous ulcers, as a cream in eczema, taken orally in ulcerative colitis and food allergies, as the powder directly applied to crural ulcers, and in solution applied to the interior of the nose and the conjunctiva in allergic conditions.

b. The use of diosmin in a cream for the treatment of crural ulcers, varicose veins and chronic venous insufficiency.

## Claims

1. Use of pharmacologically acceptable benzopyrone drugs in the manufacture of pharmaceutical formulations for topical treatment of high protein oedemas by application to the skin or mucous membranes.

2. The use of claim 1, wherein the composition is a paint, cream, powder or solution composition for application to the skin or mucous membranes.

3. The use of claim 1 or 2, wherein said benzopyrone is selected from coumarin, 7-hydroxy derivatives of coumarin, rutin, troxerutin and diosmin.

4. The use of claim 1, wherein the composition is in the form of a paint or ointment comprising a solution of coumarin in polyethylene glycol in a concentration in the range of 2.5% to 20% by weight, preferably 10% by weight.

5. The use of claim 1, wherein the composition is in the form of a cream comprising 2.5% to 90% by weight, preferably 20% by weight of 7-hydroxy coumarin in an inert cream base.

## Revendications

1. Utilisation de drogues du type benzopyrone, pharmacologiquement acceptables, pour la fabrication de formulations pharmaceutiques destinées au traitement local d'oedèmes à forte teneur en protéines, par application sur la peau ou sur les muqueuses.

2. Utilisation selon la revendication 1, dans laquelle la composition est une composition sous forme de teinture, de crème, de poudre ou de solution, destinée à l'application sur la peau ou sur les muqueuses.

3. Utilisation selon la revendication 1 ou 2, dans laquelle ladite benzopyrone est choisie parmi la coumarine, les dérivés 7-hydroxylés de la coumarine, la rutine, la troxérutine et la diosmine.

4. Utilisation selon la revendication 1, dans laquelle la composition est sous la forme d'une teinture ou d'un onguent comprenant une solution de coumarine dans le polyéthylèneglycol, de concentration comprise dans l'intervalle allant de 2,5 % à 20 % en poids, de préférence de 10 % en poids.

5. Utilisation selon la revendication 1, dans laquelle la composition est sous la forme d'une crème comprenant 2,5 % à 90 % en poids, de préférence 20 % en poids de 7-hydroxy-coumarine, dans une base de crème inerte.

## Patentansprüche

1. Verwendung eines pharmakologisch akzeptablen Benzopyron-Heilmittels zur Herstellung von pharmazeutischen Zusammensetzungen zur aktuellen Behandlung von Ödemen mit hohem Proteingehalt durch Aufbringen auf die Haut oder Schleimhäute.

2. Die Verwendung nach Anspruch 1, bei der die Zusammensetzung eine Farbe, Creme, Puder oder eine Lösungszusammensetzung zum Aufbringen auf die Haut oder Schleimhäute ist.

3. Die Verwendung nach Anspruch 1 oder 2, bei der das Benzopyron ausgewählt ist aus der Gruppe Cumarin, 7-Hydroxyderivaten des Cumarins, Rutin, Troxerutin und Diosmin.

4. Die Verwendung nach Anspruch 1, bei der die Zusammensetzung in der Form einer Farbe oder Salbe eine Lösung aus Cumarin in Polyethylenglucol in einer Konzentration in dem Bereich von 2,5 bis 20 Gew.-%, vorzugsweise 10 Gew.-%, enthält.

5. Die Verwendung nach Anspruch 1, bei der die Zusammensetzung in Form einer Creme 2,5 bis 90 Gew.-%, vorzugsweise 20 Gew.-% von 7-Hydroxycumarin in einer inerten Creme-

grundlage enthält.